# EUROPEAN PATENT SPECIFICATION

(11) **EP 0 656 906 B1**
(45) Date of publication and mention of the grant of the patent: **08.09.1999**
(21) Application number: 93913035.7
(22) Date of filing: 09.06.1993
(51) Int. Cl.: C12N 5/20, C07K 14/435, C07K 14/705, C07K 16/28, C12P 21/08, A61K 38/17, A61K 39/395, G01N 33/577

(54) **A NOVEL ENDOTHELIAL CELL MOLECULE MEDIATING LYMPHOCYTE BINDING IN MAN**
NEUARTIGES ENDOTHELZELL-MOLEKÜL, DAS DIE BINDUNG VON LYMPHOZYTEN IM MENSCHEN VERMITTELT
NOUVELLE MOLECULE DE CELLULE ENDOTHELIALE SERVANT DE MEDIATEUR A LA FIXATION DES LYMPHOCYTES CHEZ L'HOMME

(30) Priority: 09.06.1992 US 895354
(43) Date of publication of application: 14.06.1995
(73) Proprietor: Oy Biotie Therapies, 20520 TURKU (FI)
(72) Inventor: Jalkanen, Sirpa, 20760 Piispanristi (FI); Salmi, Marko, 20500 Turku (FI)
(74) Representative: VOSSIUS & PARTNER
(86) International application number: FI9300250
(87) International publication number: WO9325582

(56) References cited:
- JOURNAL OF CELLULAR BIOCHEMISTRY vol. SUP 0, no. 16F, 1992, NEW YORK, USA page 107 S. JALKANEN ET AL. 'A novel endothelial cell antigen involved in lymphocyte binding to high endothelial venules.'
- JOURNAL OF CELL BIOLOGY vol. 113, no. 5, June 1991, NEW YORK, USA pages 1213 - 1221 Y. IMAI ET AL. 'Identification of a carbohydrate-based endothelial ligand for a lymphocyte homing receptor.'
- SCIENCE vol. 257, no. 5075, 4 September 1992, WASHINGTON DC, USA pages 1407 - 1409 M. SALMI ET AL. 'A 90-kilodalton endothelial cell molecule mediating lymphocyte binding in humans.'

## Description

The present invention is directed to a novel human endothelial cell adhesion antigen, designated VAP-1, a monoclonal antibody, 1B2, that recognizes the VAP-1 antigen, and to the use of such molecules in diagnosing and treating chronic and acute inflammatory and infectious conditions characterized by lymphocyte migration.

Most mature lymphocytes continuously recirculate between the blood and lymphatic organs (Butcher, E. C., *Curr. Top. Microbiol. Immunol.* **128**, 85 (1986)). Lymphocytes leave the blood by recognizing and binding to the vascular endothelial cells. Thereafter, they migrate between the endothelial cells into the surrounding tissues. Lymphocyte trafficking allows the full repertoire of lymphocyte specificities to be available for immune reactions throughout the body; and it also facilitates the cell-cell interactions required for the generation and control of immune responses. Lymphocyte adherence to endothelial cells is dependent on interactions between complementary adhesion molecules expressed on both cell types (Springer, T. A., *Nature* **346**,425 (1990); Stoolman, L. M., *Cell* **56**, 907 (1989); Osborn, L., *Cell.* **62**, 3 (1990); Pober and Cotran, *Transplantation* **50**, 537 (1990); Butcher, E.C., *Cell* **67,** 1033 (1991)). Under normal conditions, lymphocytes mainly bind to specialized postcapillary venules called high endothelial venules (HEV). Functionally separate lymphocyte-HEV recognition systems mediating lymphocyte migration to peripheral lymph nodes, mucosal lymphoid organs, synovium and skin in an organ-specific manner have been described (Butcher, E.C., *et al., Eur. J. Immunol.* **10**, 210 (1980); Jalkanen, S., *et al., Science* **233**, 556 (1986); Picker, L. J., *et al.*, Nature **349**, 796 (1991)). In inflammation, activation of the endothelial cell results in changes of its adhesion molecule status, which largely determines the magnitude and type of leukocyte influx into the affected tissue. Thus, endothelial cell molecules are a key element in controlling the characteristics of local immune response, and obviously, a detailed understanding of the mechanisms regulating lymphocyte traffic and leukocyte extravasation can provide new means to clinically manipulate the inflammatory response.

Since in man the endothelial cell ligands mediating tissue-selective lymphocyte homing are largely unknown, a need exists for the identification of such molecules.

Two carbohydrate-based endothelial ligands for lymphocyte homing receptors with molecular weights of approximately 50 kD and 90 kD, respectively, have previously been identified (Imai et al., J. Cell Biol. 113, 1213 (1991)).

The present inventors presented in an abstract at a meeting of the European Federation of Immunological Societies in June 1991 some preliminary findings indicating the existence of a previously unknown endothelial cell antigen involved in lymphocyte binding to high endothelial venules (Salmi et al., EFIS 11th Meeting Abstracts, 21-12; 1991)

Furthermore, the present inventors presented in an abstract an endothelial cell antigen involved in lymphocyte binding to high endothelial venules, which is equivalent to the VAP-1 protein presented in the present invention. This abstract, however, does not involve an enabling disclosure in order to inevitably result in said antigen (Jalkanen et al., J. Cell. Biochem. Suppl. 0, Abstract W610, p. 107 (1992)).

Recognizing the importance of controlling inflammation, and cognizant of the need to understand the endotelial cell ligands that mediate tissue-selective lymphocyte homing, the inventors attempted to identify such molecules as expressed by human synovial vessels. These studies have culminated in the identification of a novel endothelial cell molecule that mediates lymphocyte binding in man, VAP-1, and the production of monoclonal antibodies against the same. These antibodies are useful in assays for the quantitative and qualitative assessment of VAP-1 levels, and in clinical treatments designed to antagonize VAP-1 action in a patient in need of such treatment.

Accordingly, the invention is first directed to a substantially purified and isolated human endothelial vascular adhesion protein-1 (VAP-1) having a molecular weight of 90 kDa as determined by SDS-PAGE under reducing conditions and being recognizable by the monoclonal antibody obtainable by the hybridoma cell line having the accession number DSM ACC 2041.

The VAP-1 protein is essentially free of natural contaminants that associate with such VAP-1 protein in the human or animal host.

The invention is further directed to isolated VAP-1 protein.

The invention is further directed to antibodies against the VAP-1 protein, especially monoclonal antibodies, or an anti-VAP-1 binding fragment thereof, and compositions containing such antibodies. The monoclonal antibody 1B2 is provided by this invention as well as the hybridoma cell line which produces it (DSM ACC2041).

The invention is further directed to a method for antagonizing VAP-1-mediated binding of endothelial cells to lymphocytes, said method comprising inhibiting in vitro the VAP-1-mediated lymphocyte-endothelial cell adhesion reaction by providing amounts of an anti-VPA-1 antibody sufficient to block the VAP-1 endothelial cell sites that participate in such reaction, especially where such lymphocyte-endothelial cell adhesion reaction is associated with a disease such as inflammation (chronic or acute), arthritis, rheumatoid arthritis, infection, dermatosis, inflammatory bowel disease, and autoimmune disease.

The invention is further directed to a method of diagnosing a medical condition that is mediated by VAP-1-mediated binding of endothelial cells to lymphocytes in a subject, said method comprising detecting anti-VAP-1 antibody binding to VAP-1 positive cells taken from such subject, and diagnosing said medical condition on the basis of such binding, such medical conditions including inflammation (chronic or acute), arthritis, rheumatoid arthritis, infections, dermatoses, inflammatory bowel diseases, and autoimmune diseases.

The invention is further directed to pharmaceutical composition for use in inhibiting the VAP-1-mediated lymphocyte-endothelial cell adhesion reaction, comprising an anti-VAP-1 antibody or an anti-VAP-1 binding fragment thereof and, optionally, a pharmaceutically acceptable carrier.

The invention is further directed to a diagnostic composition for use in diagnosing VAP-1-mediated binding of endothelial cells to lymphocytes in a patient, comprising an anti-VAP-1 antibody or an anti-VAP-1 binding fragment thereof and, optionally, a carrier.

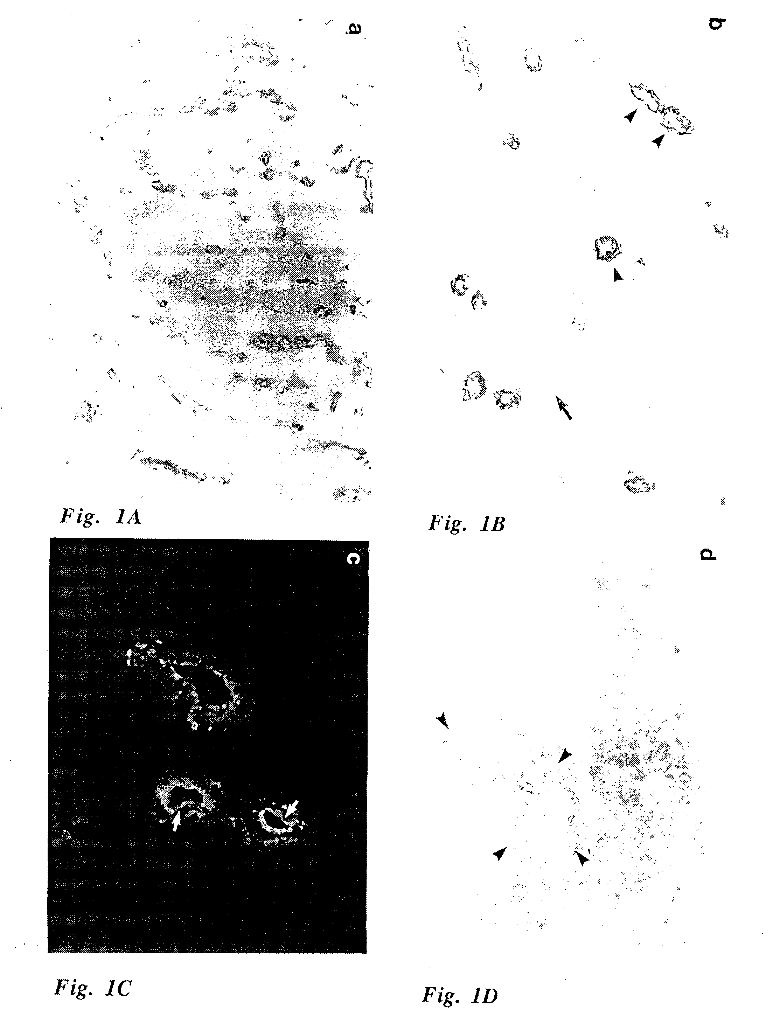

**Fig. 1**. Distribution of VAP-1 in human tissues. (**A**) In inflamed synovial membrane, mAb 182 strongly stains HEV-like vessels. (**B**) In tonsil, expression of VAP-1 in HEV varies from intense (arrowheads) to very weak (arrows) or negative. (**C**) Immunofluorescence staining of a tonsil shows the prominent expression of VAP-1 on the luminal surface of the vessels (arrows). (**D**) In appendix, only few weakly staining HEV are seen (arrowheads). Magnifications: **A** is x100, **B** is x250, **C** and **D** are x400.

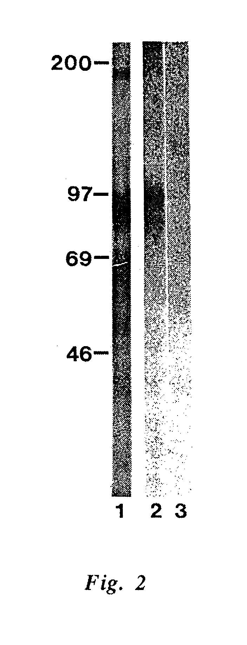

**Fig. 2**. VAP-1 is a 90 kD protein. Lane 1: silver staining of immunopurified VAP-1. Lanes 2-3: ¹²⁵1-labeled stromal cells of tonsil were immunoprecipitated with either mAb 1B2 (lane 2) or control mAb 3G6 (lane 3). The bands in the 180-200 kD area are not always present. Molecular weight standards are indicated on the left. Lymphocyte depleted tonsillar extracts were solubilized in lysis buffer (150 mM NaCI, 10 mM Tris-base, 0.15 mM MgCl₂, 1% NP-40, 1 mM PMSF and 1% aprotinin) overnight at 4°C. The lysate was centrifuged at 10000 g for 30 min at 4°C. The supernatant was precleared by passing the lysate over a Sepharose CL-4B (Pharmacia, Sweden) column. Then it was sequentially applied to three CNBr-activated Sepharose-4B (Pharmacia) columns derivatized with normal mouse serum, with irrelevant IgG₁ mAb and with 1B2 mAb (5 mg/ml, 5 ml column volume). The column was washed extensively wiht the lysis buffer. Thereafter, the material bound to the 1B2 column was eluted with 50 mM triethanolamine, lyophilized, resolved is SDS-PAGE (7.5%, reduced) and visualized using silver staining.

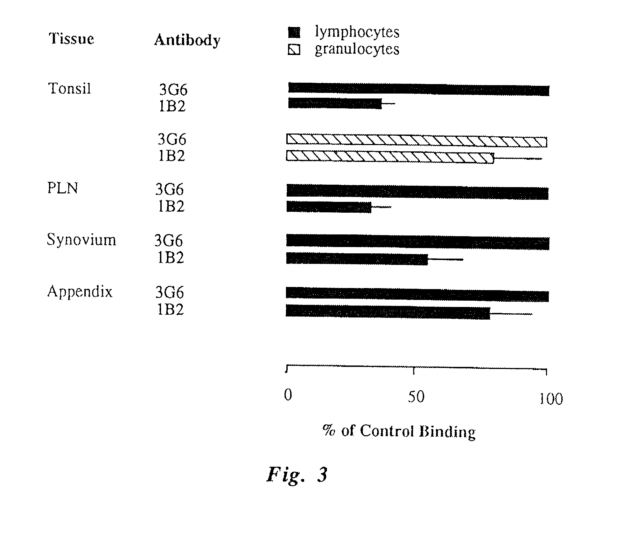

**Fig. 3**. VAP-1 is involved in lymphocyte binding to HEV. Binding of lymphocytes to tonsil, peripheral lymph node (PLN), synovial, and appendix HEV and binding of granulocytes to tonsil HEV were assessed in the presence and absence of mAb 1B2 using the *in vitro* frozen section assay. Results of three independent experiments are presented as percentages of control binding with standard errors (100%= number of bound cells on 3G6 treated sections).

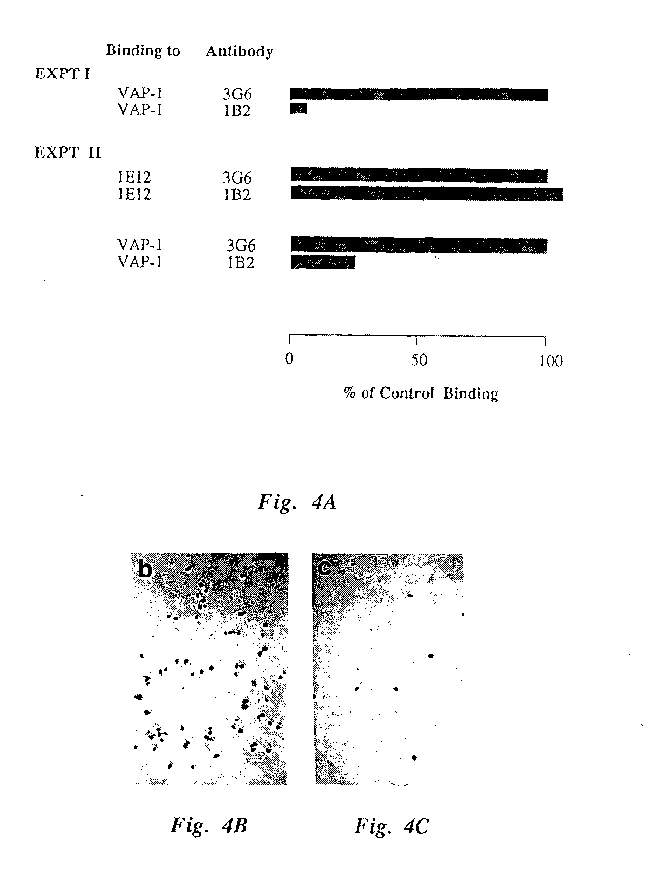

**Fig. 4**. Isolated VAP-1 supports lymphocyte binding. Immunopurified VAP-1 and control proteins (1E12; an unrelated endothelial cell molecule that supports lymphocyte binding, and BSA) were absorbed on glass, and lymphocyte binding was determined. (**A**) Results from two independent experiments are presented as percentages from control binding (100%= number of cells bound to plate-bound VAP-1 or 1E12 after mAb 3G6 treatment). Nonspecific background (binding to BSA) is subtracted from all analyses. (**B**) Lymphocyte binding to VAP-1-coated well in the presence of mAb 3G6. (**C**) Lymphocyte binding to VAP-1-coated well in the presence of mAb 1B2. VAP-1 and 1 E12 were affinity purified from tonsillar extracts as indicated in Figure 2. Purified VAP-1, 1E12 and heat-inactivated BSA were diluted in 20 mM Tris-HCI, pH 7.4, 150 mM NaCI, 2 mM MgCl₂, 2 mM CaCl₂ with 0.01% β-octyl glucopyranoside as detergent. Proteins were absorbed onto glass wells (Lab-Tek chamber slides, Nunc) for 16 h at +4°C. After blocking in PBS containing 1 mg/ml BSA for 30 min at room temperature, 1B2 or 3G6 supernatants were added into wells and incubation was continued for 30 min at room temperature. Meanwhile, freshly isolated peripheral blood mononuclear cells were incubated in RPMI 1640 containing 10% FCS and 10 mM Hepes for 1 hour at 37°C in tissue culture bottles to deplete the plastic adherent monocytes. Non-adherent lymphocytes (1.8x10⁶ cells/well) in 100 µl RPMI 1640 were applied into each well. After 30 min incubation at 37°C, the non-adherent cells were removed by flicking. The tops of the wells were removed, the slides were washed by gentle stream of PBS, and fixed in cold PBS containing 1 % glutaraldehyde. Thereafter, the cells were stained using the Diff-Quick stains. The bound cells were quantitated by visually scoring the number of cells in each well (total area of 50 mm²/sample).

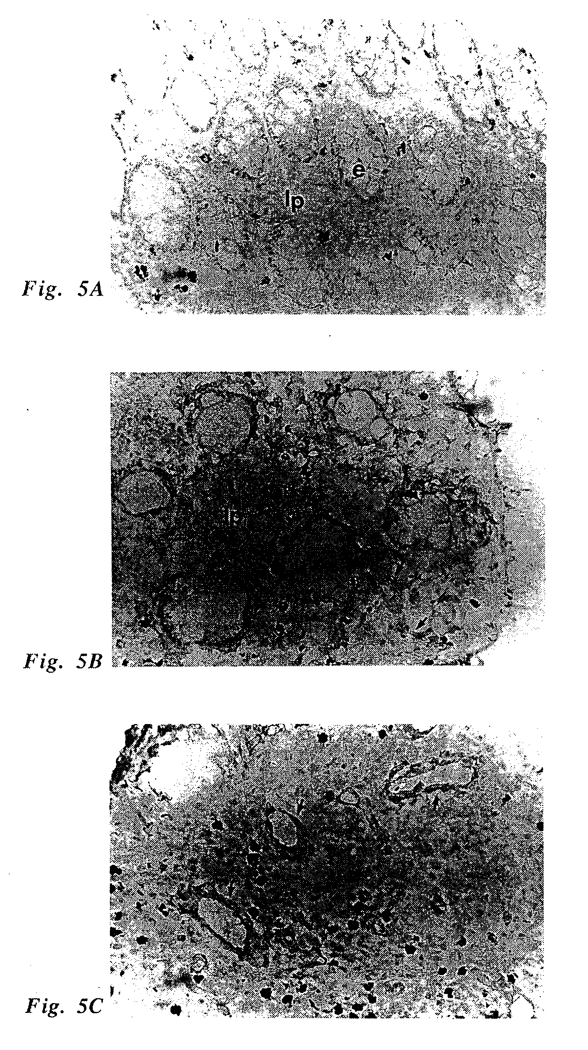

**Fig. 5**. VAP-1 is up-regulated in the inflamed gut. In normal gut, only a few faintly positive vessels in the lamina propria are observed (A, in this area, venules are practically negative for VAP-1). In the inflamed gut (ulcerative colitis), numerous VAP-1 positive venules (arrows) are seen both in B) lamina propria and in C) organized lymphoid follicles. Endogenous peroxidase containing cells (mast cells) show non-specific reactivity. e, epithelial cells of the gut; Ip, lamina propria. Magnification x250.

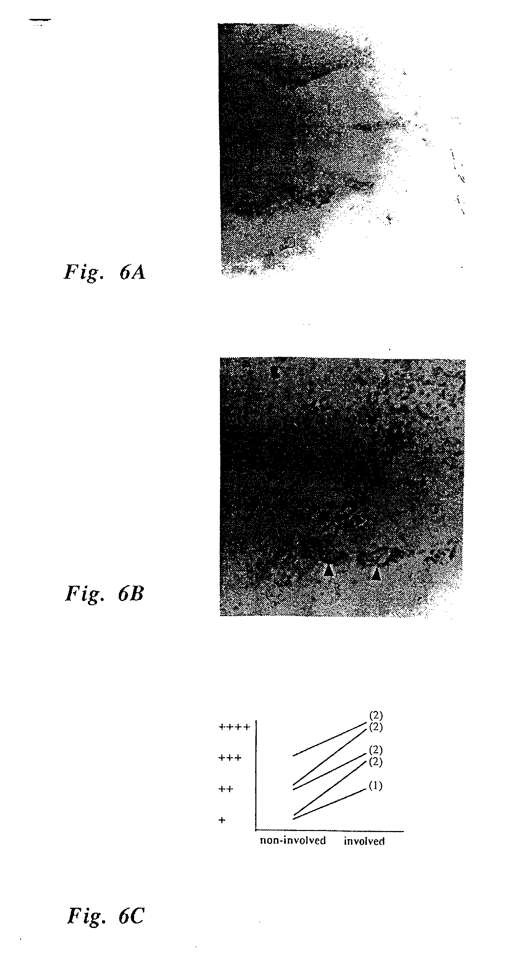

**Fig. 6.** VAP-1 induction in chronic dermatoses. Skin biopsies from normal area (A) and from psoriatic lesion (B) of the same patient reveal induction of VAP-1 in dermal vessels (arrowheads) at sites of inflammation. Perivascular leukocyte infiltrates can be seen around VAP-1 positive venules. e, epidermis. Magnification x200. C) Expression of VAP-1 (scored from + to ++++) in normal and diseased skin was determined in the paired biopsies (non-involved and involved) from the same patients. In the parenthesis is shown the number of patients belonging to each group.

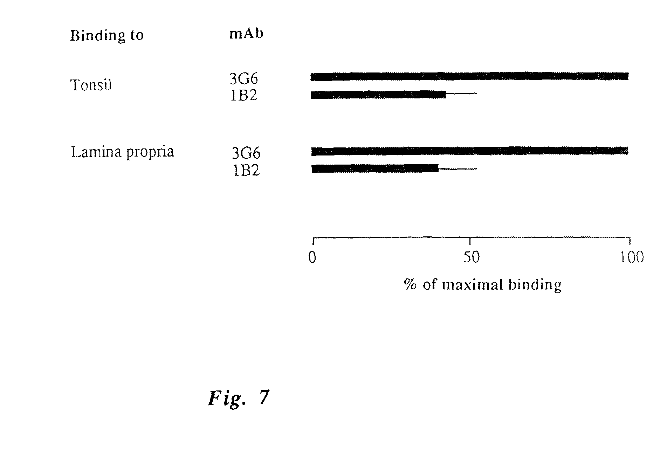

**Fig. 7**. The inflammation-induced VAP-1 mediates lymphocyte binding. A frozen section binging assay was performed, in which binding of PBL to Factor VII positive venules in inflamed lamina propria was analysed. Inhibition assays were done by preincubating tissue sections with mAbs 1B2 and 3G6. Results are presented as percentages of control binding with standard errors (i.e., binding of PBL in the prescence of mAb 3G6 defines 100% binding).

Interactions between leukocyte surface receptors and their ligands on vascular endothelial cells critically control lymphocyte traffic between the blood and various lymphoid organs. as well as extravasation of leukocytes into sites of inflammation. We describe nere a novel 90 kD human endothelial cell adhesion molecule (VAP-1) defined by a monoclonal antibody 1B2. VAP-1 is preferentially expressed on synovial, peripheral lymph node and tonsil high endothelial venules (HEV) and 1B2 markedly inhibits lymphocyte binding to HEV in these tissues, as opposed to those at mucosal sites. Moreover, lymphocytes bind to immunoaffinity-isolated VAP-1 in an 1B2-inhibitable manner. The expression pattern, molecular weight, and functional properties define VAP-1 as a new endothelial ligand for lymphocytes. We conclude that VAP-1 is a novel vascular molecule directly involved in lymphocyte trafficking in man.

### Production of Antibodies

For the initial identification of VAP-1 protein, monoclonal antibodies against synovial vessels were produced by immunizing an appropriate animal, such as mice, with stromal elements of human synovium. Such stromal elements of human synovium may be obtained using methods known in the art. Synovial stroma may be isolated by depleting the lymphocytes from the synovial tissue. The synovial tissue is minced and the minced tissue pressed through a stainless steel screen. The stromal elements are collected from the top of the screen.

The immunogen is prefereably administered together with an adjuvant, such as, for example, incomplete Freund's adjuvant; however, any appropriate adjuvant may be used. The immunogen and adjuvant are injected by any regime or protocol that will result in the induction of antibodies synthesis. For example, injection of the immunogen (synovial stromal elements containing approximately 1 µg VAP-1 antigen per injection) three times at one week intervals, into the footpads of specific-pathogen free Balb/c mice, will induce the immune response.

Lymphocytes from popliteal lymph nodes are isolated using methods known in the art as described above, by pressing the minced lymph nodes through a stainless steel screen and collecting the released lymphocytes from the flow through, and fused with the nonsecreting NS-1 mouse myeloma cells (available from ATCC, No. TIB 18) using standard protocols. Hybridomas may be screened using any appropriate method, such as immunoperoxidase staining of frozen sections. One hybridoma (1B2, subclass IgG₁) that produced an antibody reactive with vascular endothelium of synovium was cloned twice by limiting dilution and was chosen for further analysis. The antigen recognized by mAb 1B2 was named VAP-1 (for Vascular Adhesion Protein-1).

Antibodies used in the methods of the invention as VAP-1 binding proteins are preferably monospecific antibodies, that is, antibodies with a specificity against only VAP-1, or an antigenic fragment thereof. Monospecific antibodies may be both polyclonal and monoclonal.

Polyclonal antibodies may be prepared by injecting a suitable animal with a substantially pure preparation of VAP-1 followed by one or more booster injections at suitable intervals.

It is, however, preferred to employ monoclonal antibodies (or biologically active derivatives thereof such as Fab', F(ab')₂ or Fv fragments), directed against VAP-1 antigen, in the methods of the invention. It should be noted that the monoclonal antibodies may be from any suitable source, and may thus be selected from, for instance, murine or human monoclonal antibodies.

The antibody may also be produced by cloning a DNA sequence coding for the antibody or a biologically active derivative thereof into suitable cell, e.g., a microbial, plant, animal or human cell, and culturing the cell under conditions conductive to the production of the antibody or biologically active derivative in question and recovering the antibody or biologically active derivative thereof from the culture.

The antibodies used in the reagent of the invention should preferably be in substantially pure form in order to improve the accuracy of the method.

### Characteristics of VAP-1

Isolated VAP-1 has a molecular weight of 90 kD under reducing conditions, and 100 kD under non-reducing conditions.

To isolate VAP-1 antigen, a lymphocyte-depleted tonsilar extract is the preferred source of the VAP-1 protein. These extracts are prepared by depleting the lymphocytes from tonsilar tissue by pressing the minced tissue through a stainless steel screen. The stromal elements are collected from the top of the screen. However, any cell type that expressed VAP-1 may be used in the procedure below as the exemplified procedure relies on the affinity of VAP-1 for 1B2 mAb for the final isolation.

All steps should be performed at refrigerated temperatures (about 4°C). The cells are gently lysed (for example overnight at 4°C) in an buffer that contains agents for the inhibition of proteolysis, such as a buffer containing 150 mM NaCI, 10 mM Tris-base, 0.15 mM MgCl₂, 1 % NP40, 1mM PMSF and 1 % aprotinin. Such extracts are preferably then cleared of cell lysis debris by a mild centrifugation at, for example, 10,000 g for 30 min. The supernatant is then applied to a series of affinity columns that provide as the affinity agent, in succession, (1) normal mouse serum, (2) non-specific IgG₁ mAb (such as 1E12 or any commercially available non-specific IgG₁ mAb) and (3) 1B2 mAb. Material bound to the 1B2 mAb column is eluted with 50 mM triethanolamine, and lyophilized. Using this approach, VAP-1 is isolated from tonsillar stroma. Other equivalent methods known in the art may be used.

As detected using monoclonal antibody 1B2, in cells, VAP-1 is abundant in HEV-like venules in inflamed synovial membranes. VAP-1 is not present in infiltrating leukocytes or in any connective tissue component of the synovial stroma. In peripheral lymph node and tonsil, VAP-1 appears to be present in the majority of HEV. VAP-1 is highly localized at the luminal side of the endothelial cells. A granular staining of VAP-1 is seen in the endothelial ceil cytoplasm, and also at the abluminal surface.

Especially in tonsil, VAP-1 levels greatly vary between different HEV, and few individual HEV (with a typical plump morphology) completely lack VAP-1. In appendix and in lamina propria of the gut, only few faintly staining venules appear to be present. Weak expression of VAP-1 is found on dendritic-like cells in germinal centers and on smooth muscle cells of arteries, veins and bowel wall.

In contrast, VAP-1 is practically absent from the luminal surface of larger vessels. In addition to leukocytes in tissue sections, the following cells do not appear to express VAP-1: peripheral blood lymphocytes, monocytes, NK cells, granulocytes and isolated tonsillar leukocytes, the T-lymphoblastoid cell line CCRF-CEM (CCL 119, ATCC); B-lymphoblastoid cell lines KCA and IBW-4 (EBV transformed B cell lines originally obtained from E.Engleman, Stanford University), a monocytic cell line U937 (CRL 1593, ATCC); and leukemic cell lines KG-1 (CCL 246, ATCC); KG-1a (CCL 246.1, ATCC) and K562 (CCL 243, ATCC); the endothelial cell line EaHy-926 (a hybridoma cell line between carcinoma and endothelian cells, Edgell *et al., Proc. Natl. Acad. Sci. USA 80:3724 (1983));* and primary cultures of smooth muscle cells, fibroblasts and keratinocytes, and the epithelioid HeLa cell line (CCL 2, ATCC). Human umbilical vein endothelial cells (HUVEC), isolated by the method of Jaffe (*J. Din. Invest.52:*2745 (1973)) did not express VAP-1 either basally or after 4 h or 20 h treatments with IL-1 (20,100 U/ml), TNF (200 U/ml) or LPS (0.1,1.0 µg/ml).

Comparison of VAP-1 with the known endothelial cell molecules mediating leukocyte binding reveals several differences. Intercellular adhesion molecules -1 and -2 (ICAM-1 and ICAM-2), vascular cell adhesion molecule-1 (VCAM-1), E-selectin (ELAM-1) and P-selectin (CD62, PADGEM, GMP 140) are all expressed on HUVEC either basally or after induction by inflammatory mediators (Springer, T.A., *Nature* 346:425 (1990); Stoolman, L. M., *Cell 56*:907 (1989); Osborn, L., *Cell 62.3* (1990); Pober and Cotran, *Transplantation50:*537 (1990); Butcher, E.C., *Cell67:* 1033 (1991); de Fougerolles, A.R., *et al., J. Exp. Med.* **174**, 253 (1991); Osborn L., *et al., Cell* **59,** 1203 (1989); Bevilacqua, M.P., *et al., Proc. Natl. Acad. Sci.* **84,** 9238 (1987); McEver, R. P., *et al., J. Clin. Invest. 84,* 92 (1989); Hattori, R., *et al., J. Biol. Chem.* **264**, 7768 (1989); Wellicome, S.M., *et al., J. Immunol.* **144,** 2558 (1990); Pober, J. S., *et al., J. Immunol.* **137**, 1893 (1986); Dustin, M. L., *et al., J. Immunol.* **137,** 245 (1986)). In contrast, VAP-1 is neither constitutively expressed nor inducible by IL-1, TNFa or LPS treatments on the surface or in the cytoplasm of HUVEC. Tissue distributions of these molecules are clearly distinct - also, the distribution is distinct when analyzed on parallel sections of tonsil (data not shown). ICAMs stain the luminal surface of most large and small vessels and certain leukocytes (de Fougerolles, A. R., *et al., J. Exp. Med.* **174,** 253 (1991); Dustin, M. L., *et al., J. Immunol.* **137**, 245 (1986)). VCAM-1 and ELAM-1, on the other hand, only stain a few venules in inflamed tissues (Rice, G. E., *et al., J. Exp. Med.* **171**, 1369 (1990); Rice, G. E., *et al., Am. J. Patriot,* **138**, 385 (1991); Cotran, R. S., *et al*., *J. Exp. Med.* **164,** 661 (1986)). Instead, VAP-1 is strongly expressed on the vast majority of HEV at non-mucosal sites, and it is absent from all white cells and cell lines tested. The molecular weights of the known adhesion molecules are also clearly different from that of VAP-1, with the exception of ICAM-1 (ICAM-2 is a 60 kD, VCAM-1 110 kD, E-selectin 115 kD and P-selectin 140 kD molecule, Stoolman, L.M., *Cell, 56:* 907 (1989); Osborn, L., *Dell 62:*3 (1990); and de Fougerolles, A. R., *et al., J. Exp. Med.* **174**, 253 (1991)). Furthermore, VAP-1 is mainly involved in lymphocyte binding, while (CAMs, E- and P-selectin also efficiently mediate adhesion of polymorphonuclear leukocytes (Springer, T.A., *Nature* **346**, 425 (1990); Stoolman, L.M., *Cell* **56,** 907 (1989); Osborn, L, *Cell.* **62,** 3 (1990); Pober and Cotran, *Transplantation* **50**, 537 (1990); Butcher, E.C., *Cell* **67**, 1033 (1991)). The only endothelial adhesion molecule described so far that is involved in lymphocyte binding in man and is not expressed on HUVEC is the MECA-79-defined antigen (Berg, E.L., *et al., J. Cell Biol,* **114,** 343 (1991)). However, it is a tissue-specific addressin of peripheral lymph nodes. Moreover, VAP-1 is not co-expressed in all MECA-79-positive venules, and mAb 1B2 does not recognize purified MECA-79 antigen.

Therefore, the expression pattern, function and molecular weight of VAP-1 indicate that it is not identical with any of the previously defined endothelial molecules involved in lymphocyte binding and that the degree of inflammation correlates to the level of VAP-1 expression in vivo. These results show that VAP-1 is relevant to understanding of the physiologic lymphocyte recirculation in man, and is especially valuable for dissecting the molecular mechanisms of tissue selective lymphocyte homing.

### Uses of VAP-1 and VAP-1 Binding Compounds

VAP-1 binding compounds as comprised by the present invention are anti-VAP-1 antibodies or anti-VAP-1 binding fragments thereof. Other possible VAP-1 binding compounds may be suitable to be used for the same purposes as indicated below for the VAP-1 binding compounds as comprised by the present invention. However, neither such other compounds nor their uses for the purposes as indicated below are included as embodiments of the present invention.

The present invention relates to a diagnostic reagent for the detection of VAP-1 protein and VAP-1 positive cells, in samples taken from the human or animal body. Such reagent may be an antibody reactive with VAP-1 protein, or a VAP-1-reactive fragment of said antibody, labelled, if desired, with a substance which permits the detection of binding of the antibody to the isolated VAP-1, or cells that express VAP-1 on their surface. Such diagnostic composition may be provided in a kit, such kit providing, in separate containers,
(a) an antibody reactive with VAP-1, or a biologically active derivative of said antibody, preferably labelled with a substance which permits detection of binding of the antibody to VAP-1 antigen: and
(b) purified VAP-1 protein, to provide a standard for evaluation of the assay results.

In another aspect, the present invention is directed to a pharmaceutical composition comprising efficacious levels of a VAP-1-binding compound (which is an antibody reactive with VAP-1, or a biologically active derivative of such antibody for lessening or treating inflammation in the human or animal body, by administering to a human or animal patient in need of such treatment.

The term "treatment" or "treating" is intended to include the administration of VAP-1-binding compounds to a subject for purposes which may include prophylaxis, amelioration, prevention or cure of disorders mediated by VAP-1 adhesion events. The particular VAP-1-binding molecules that are the subject of the methods of the invention are purified native and recombinant VAP-1-binding proteins, which are antibodies or anti-VAP-1 binding fragments thereof.

For administration to a patient, the reagent of the invention may be formulated in any manner which makes it suitable for parenteral, nasal, enteric or rectal administration. Thus, the reagent may be in the form of, for instance, an injectable formulation, aerosol formulation, suspension, solution, enema, etc. The reagent may be formulated with pharmaceutically acceptable excipients or vehicles, e.g., isotonic saline, in accordance with conventional pharmaceutical practice. The dosage level of the reagent will be sufficient to provide an anti-inflammatory effect by the blocking of VAP-1 adhesion events in the patient.

The reagent of the invention is suitable for diagnosing or treating any condition involving a VAP-1 adhesion-mediated increased inflammatory reaction. Thus, the reagent is useful for diagnosing such conditions as arthritis, local infections, dermatoses, inflammatory bowel diseases, autoimmune diseases, etc.

In one embodiment, efficacious levels of the VAP-1-binding compound as defined above are for administration so as to provide therapeutic benefits against the secondary harmful inflammatory effects of inflammation. By an "efficacious level" of the VAP-1-binding compound is meant a level in which the toxic effects of VAP-1 mediated events are, at a minimum, ameliorated. By "excessive" host VAP-1-mediated events are meant an level of VAP-1 mediated adhesion events in the subjects which exceeds the norm for the healthy medical state of the subject. By secondary" tissue damage or toxic effects is meant the tissue damage or toxic effects which occur to otherwise healthy tissues, organs, and the cells therein, due to the presence of excessive VAP-1-mediated adhesion events, including as a result of a "primary" stimulus elsewhere in the body.

Infusion of the VAP-1-binding compounds as defined above, such as, for example, VAP-1 antibodies, into a patient, results in a binding of such antibodies to the patient's cells that express VAP-1 on their surface, such as synovial HEV, peripheral lymph node HEV and tonsil HEV so as to prevent their adhesion to other cells by VAP-1 binding, thus preventing or inhibiting lymphocyte adherence to such tissues and cells, and thus preventing undesired lymphocyte trafficking or influx into the affected tissues or cells, thus preventing undesired inflammatory responses that arose from VAP-1 directed leukocyte trafficking and leukocyte extravasation.

Accordingly, the pharmaceutical compositions of the invention provide for compositions containing VAP-1 binding compounds as defined above, in amounts sufficient to antagonize (fully or partially) the patient's native VAP-1 binding to biological targets of VAP-1 in such patient, and specifically to endothelial cells.

VAP-1 binding compounds may be conjugated, either chemically or by genetic engineering, to fragments of other agents which provide a targeting of such VAP-1-binding compounds as defined above to a desired site of action. Alternatively, other compounds may be conjugated, either chemically or by genetic engineering, to the VAP-1-binding compound as defined above, so as to enhance or provide additional properties to such VAP-1-binding compound, especially properties which enhance the compound's ability to promote relief of VAP-1 adhesion-mediated toxic effects.

Amounts and regimens for the administration of VAP-1-binding compounds as defined above can be determined readily by those with ordinary skill in the clinical art of treating inflammation-related disorders such as arthritis and tissue injury. Generally, the dosage of VAP-1-binding compound treatment will vary depending upon considerations such as: type of VAP-1-binding compound employed: age; health; medical conditions being treated; kind of concurrent treatment, if any, frequency of treatment and the nature of the effect desired; extent of tissue damage; gender; duration of the symptoms; and counterindications, if any, and other variables to be adjusted by the individual physician. A desired dosage can be used to be administered in one or more applications to obtain the desired results. Pharmaceutical compositions containing the VAP-1 binding compound of the invention, such as anti-VAP-1 antibody, may be provided in unit dosage forms.

The pharmaceutical compositions containing the VAP-1 binding compounds of the invention can be used to be administered in any appropriate pharmacological carrier for administration. They can be used to administered in any form that effects prophylactic, palliative, preventative or curing conditions of VAP-1 mediated events in humans and animals. For the purpose of definition it is intended that the expression "pharmaceutical composition for treating" a disease, and like expressions, throughout the specification and claims, be taken to include a pharmaceutical composition for the prevention of such a disease.

Preparations of the VAP-1-binding proteins of the invention as defined above for parenteral administration includes sterile aqueous or non-aqueous solvents, suspensions and emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils. Intravenous vehicles include fluid and nutrient replenishers, electrolyte replenishers, such as those based upon Ringer's dextrose and the like.

The VAP-1-binding compounds of the invention as defined above may also be used to be administered by means of pumps, or in sustained-release form, especially, when the primary injury is prolonged or delayed rather an acute. An example in which the primary injury is often prolonged or delayed rather than acute is an infection or sprain wherein the damage to the tissue or muscle is not revealed (or persists) until days after the primary infection or damage. The VAP-1-binding molecules of the invention may also be delivered to specific organs in high concentration by means of suitably inserted catheters, or by providing such molecules as a part of a chimeric molecule (or complex) which is designed to target specific organs.

Administration in a sustained-release form is more convenient for the patient when repeated injections for prolonged periods of time are indicated. For example, it is desirable to use the VAP-1-binding proteins of the invention as defined above for administration in a sustained-release form when the pharmaceutical compositions of the invention are being used to treat a genetic or chronic inflammatory disease that is based upon an VAP-1-related disorder so as to maximize the comfort of the patient.

The VAP-1-binding compound of the invention can be employed in dosage forms such as tablets, capsules, powder packets, or liquid solutions for oral administration if the biological activity of the VAP-1-binding compound is not destroyed by the digestive process and if the characteristics of the compound allow is to be absorbed across the intestinal tissue.

The pharmaceutical compositions of the present invention are manufactured in a manner which is in itself know, for example, by means of conventional mixing, granulating, dragee-making, dissolving, lyophilizing or similar processes. The compositions of the present invention, in and of themselves, find utility in the control of VAP-1-induced physiological damage, be it chronic or acute. The compositions of the invention obviate the body's own mechanisms for recognizing VAP-1 adhesion to its maximum potential.

In intravenous dosage form, the compositions of the present invention have a sufficiently rapid onset of action to be useful in the acute management of potential tissue damage.

Additionally, a low potency version is useful in the management of mild or chronic VAP-1-related disorders.

In addition, the compositions of the present invention provide requisite reagents for the laboratory assay of VAP-1 levels in human's or in an animal's bloodstream or extracellular tissues.

VAP-1-binding proteins that are substantially free of natural contaminants can be isolated and purified from their natural or recombinant sources in accordance with conventional conditions and techniques in the art previously used to isolate such proteins, such as extraction, precipitation, chromatography, affinity chromatography, electrophoresis, or the like.

The following examples are merely intended to illustrate the present invention and not in any way to limit its scope.

### EXAMPLES

### Example 1

### Tissue distribution of VAP-1

The tissue distribution of VAP-1 was determined by immunoperoxidase staining of cryostat sections. The sections were incubated with primary antibodies (1B2 and 3G6, a control mouse IgG₁ mAb against chicken T cells) for 30 min. After two washings in phosphate buffered saline (PBS, 8 g NaCI, 1.21 g K₂HPO₄ and 0.34 g KH₂PO₄ per liter, pH 7.2), peroxidase-conjugated sheep anti-mouse IgG (Dakopatt, Denmark) in PBS containing 5% AB-serum was added for 30 min. Next, 3'3'-diaminobenzidine hydrochloride in PBS containing 0.03% hydrogen peroxide was used as a chromogen. After the staining, the sections were counterstained with hematoxylin. For immunofluorescence staining, 3 µm cryostat sections were overlaid with primary antibodies and FITC-conjugated sheep anti-mouse IgG (Sigma, St. Louis) was used as a second-stage reagent.

Immunohistological stainings revealed that monoclonal antibody 1B2, strongly stained HEV-like venules in inflamed synovial membranes (Fig. 1A). No staining was observed in infiltrating leukocytes nor in any connective tissue component of the synovial stroma. The antigen recognized by mAb 1B2 was named as VAP-1 (for *V*ascular *A*dhesion *P*rotein-1). In peripheral lymph node and tonsil, mAb 1B2 reacted with the majority of HEV (Fig. 1B). VAP-1 was intensely expressed at the luminal side of the endothelial cells (Fig. 1C). A granular staining was seen in the endothelial cell cytoplasm, and also the abluminal surface was mAb 1B2 positive. Especially in tonsil, the staining intensity notably varied between different HEV, and few individual HEV with a typical plump morphology were 1B2-negative (Fig. 1D). In appendix and in lamina propria of the gut, only few faintly staining venules were detected. Weak expression of VAP-1 was also seen on dendritic-like cells in germinal centers and on smooth muscle cells of arteries, veins and bowel wall. In contrast, VAP-1 was practically absent from the luminal surface of larger vessels. Like leukocytes in tissue sections, peripheral blood lymphocytes, monocytes, NK cells, granulocytes and isolated tonsillar leukocytes were all completely 1B2-negative in FACS analyses. T-lymphoblastoid (CCRF-CEM), B-lymphoblastoid (KCA, IBW-4), monocytic (U937) and leukemic (KG-1, KG-1a, K 562) cell lines all lacked VAP-1. Moreover, VAP-1 was absent from human umbilical vein endothelial cells (HUVEC) basally; further 4 h or 20 h treatments with IL-1 (20,100 U/ml), TNF-µ(200 U/ml) or LPS (0.1,1.0 µg/ml) could not induce its synthesis. An endothelial cell line (EaHy-926) was also 1B2-negative. Primary cultures of smooth muscle cells, fibroblasts and keratinocytes, and an epithelioid (HeLa) cell line did not express VAP-1.

### Example 2

### Intracellular localization of VAP-1

In addition to the tissue localization experiments described in Example 1, the intracellular localization of VAP-1 was further studied by confocal microscopy of thick sections (15 µm) cut from tonsils. Sections were incubated with mAb 1B2 or 3G6 fro 15 min, washed twice with PBS and overlaid with FITC-conjugated sheep anti-mouse 1g for another 15 min. Thereafter, samples were mounted in glycerol containing 10% PBS and phenylendiamine as anti-fadeing agent prior to analysis by confocal microscope. By microscopic inspection it was readily discernible that VAP-1 is present on the luminal surface of HEV as well as in discrete granules within the cytoplasm. The identity of these granules is currently unclear, but in two-color immunofluorescence stainings using mAb 1B2 and antibody against Factor VIII, the granules did no co-localize. Thus, VAP-1 positive granules are not Weibel-Palade bodies of endothelial cells, known to reside the endothelial cell adhesion molecule, P-selectin.

### Example 3

### Determination of the molecular weight of VAP-1

Lymphocyte-depleted tonsillar extracts were solubilized in lysis buffer (150 mM NaCI, 10 mM Tris-base, 0.15 mM MgCl₂, 1% NP40, 1 mM PMSF and 1% aprotinin) overnight at 4°C. The lysate was centrifuged at 10000 g for 30 min at 4°C. The supernatant was precleared by passing the lysate over a Sepharose CL-4B (Pharmacia, Sweden) column. Then it was sequentially applied to three CnBr-activated Sepharose-4B (Pharmacia) columns derivatized with normal mouse serum, with irrelevant IgG₁ mAb and with 1B2 mAb (5 mg/ml, 5 ml column volume). The column was washed extensively with the lysis buffer. Thereafter, the material bound to the 1B2 column was eluted with 50 mM triethanolamine, lyophilized, resolved in SDS-PAGE (7.5%, reduced) and visualized using silver staining.

For iodine labeling, lymphocyte-depleted tonsillar extracts were digested in RPMI 1640 containing 100 U/ml collagenase (type II from Clostridium histolyticum, Sigma) 10% fetal calf serum (FCS), antibiotics, and 10 mM Hepes for 1 hour at 37°C with gentle stirring. After the collagenase digestion, cells were washed in HBSS, and surface-labeled with ¹²⁵I using the lactoperoxidase method. Iodinated cells were lysed with the lysis buffer, and the lysate was clarified by centrifugation at 10000 g for 15 min. The lysate was precleared for 16 h at 4°C with CnBr-activated Sepharose coupled to normal mouse serum. Immunoprecipitations were carried out with CnBr-activated Sepharose 4B beads conjugated with mAbs 1B2 or 3G6. The samples were analyzed using 7.5 % SDS-PAGE under reducing (2-mercaptoethanol) conditions.

To determine the molecular weight of VAP-1, affinity-isolated molecule from tonsillar stroma was subjected to SDS-PAGE. Silver staining of the gel revealed a major band of apparent molecular weight of 90 kD under reducing conditions (Fig. 2). VAP-1 migrated slightly slower under non-reducing conditions (Mᵣ 100 kD). Analyses of immunoprecipitates from iodinated stromal cells of tonsil confirmed the reactivity of mAb 1B2 with a 90 kD molecule (and a slightly smaller degradation product) (Fig. 2). Also a 180-200 kD band was sometimes visible.

### Example 4

### Binding of lymphocytes to tonsil, peripheral lymph node (PLN), synovial, and appendix HEV and binding of granulocytes to tonsil HEV

The details of this technique have been described earlier (Jalkanen and Butcher, *Blood* **66,** 577 (1985)). Briefly, freshly cut frozen sections from human tonsil, synovium, appendix and peripheral lymph node were incubated with 1B2 or 3G6 supernatants for 30 min at 7°C with mild rotation. Ficoll-isolated lymphocytes (3x10⁶/section) in HBSS containing 5% FCS and 10 mM Hepes were then added and incubation was continued for 30 min. After incubation, non-adherent cells were gently tipped off and the adherent cells were fixed overnight in cold PBS containing 1% glutaraldehyde. Cells bound to HEV on four to six sections per tissue per sample were counted (minimum of 100 HEV) single blind. When determining granulocyte binding, the assay was done similarly, with the exception that granulocytes (isolated using Histopaque 1119, Sigma) were kept in Ca²⁺- g²⁺- free HBSS, until just before application onto the sections.

The tissue distribution of VAP-1 on endothelial cells *in vivo* suggested that it might function as a specific recognition element for leukocytes. Therefore, the functional role of VAP-1 in HEV-binding was studied by using the modified Stamper-Woodruff in vitro assay (Jalkanen and Butcher, *Blood* **66,** 577 (1985)). Pretreatment of the frozen sections with mAb 1B2 inhibited lymphocyte binding to HEV (Fig. 3). The inhibitory effect was most pronounced in tonsil and peripheral lymph node, but binding to synovial HEV was also significantly reduced. Lymphocyte binding to appendix HEV and granulocyte binding to tonsil HEV were less affected (Fig. 3). These findings indicate that VAP-1 either mediates or associates closely with endothelial cell elements mediating lymphocyte recognition of peripheral lymph node, tonsil and synovial HEV. To directly evaluate the involvement of VAP-1 in lymphocyte-endothelial cell interaction, binding of lymphocytes to affinity-isolated VAP-1 was analyzed (Fig. 4). Lymphocytes adhered efficiently to plate-bound VAP-1. Lymphocyte binding to VAP-1 was specifically inhibited with mAb 1B2, but not with a control mAb 3G6. MAb 1B2 did not prevent lymphocyte binding to another unrelated endothelial cell molecule (Fig. 4).

Tissue distribution and HEV-binding results suggest that VAP-1 is mainly involved in lymphocyte trafficking to peripheral lymph node, tonsil and synovium. Interestingly, in tonsil lack of VAP-1 expression defines a minor subset of postcapillary venules, which are morphologically indistinguishable from 1B2-positive ones. Since tonsils are intimately associated to the gastrointestinal tract, they may contain HEV-specificities of both mucosal (VAP-1 negative) and peripheral lymph node (VAP-1 positive) types. It remains to be determined, how the phenotypic difference in VAP-1 expression correlates to lymphocyte binding capacity of each individual HEV. The scarcity of VAP-1 in mucosal lymphoid organs implies that this endothelial antigen may be differentially regulated in distinct lymphocyte recognition systems. Moreover, the degree of inflammation correlates to the level of VAP-1 expression *in vivo.*

### Example 5

### Assay for the Effect of 1 B2 on the Binding of Cells to VAP-1

VAP-1 and 1E12 were affinity purified from tonsillar extracts as described in Example 2. Purified VAP-1, 1E12 and heat-inactivated BSA were diluted in 20 mM Tris-HCI, pH 7.4, 150 mM NaCI, 2 mM MgCl₂, 2mM CaCl₂ with 0.01% β-octyl glucopyranoside as detergent. Proteins were absorbed onto glass wells (Lab-Tek chamber slides, Nunc) for 16 h at +4°C. After blocking in PBS containing 1mg/ml BSA for 30 min at room temperature, 1B2 or 3G6 supernatants were added into wells and incubation was continued for 30 min at room temperature. Meanwhile, freshly isolated peripheral blood mononuclear cells were incubated in RPMI 1640 containing 10% FCS and 10 mM Hepes for 1 hour at 37°C in tissue culture bottles to deplete the plastic adherent monocytes. Non-adherent lymphocytes (1.8x10⁶ cells/well) in 100 µl RPMI 1640 were applied into each well. After 30 min incubation at 37°C, the non-adherent cells were removed by flicking. The tops of the wells were removed, the slides were washed by gentle stream of PBS, and fixed in cold PBS containing 1 % glutaraldehyde. Thereafter, the cells were stained using the Diff-Quick stains. The bound cells were quantitated by visually scoring the number of cells in each well (total area of 50 mm²/sample).

### Example 6

### Partial Amino Acid Sequence of VAP-1

A partial amino acid sequence of the 90 kD VAP-1 antigen has been determined. The partial amino acid sequence is 20 amino acids long as is TEDGDMXLVNGASANEGXVE [SEQ ID NO. :1:]. A search of the protein and DNA sequence databases did not reveal this sequence in any other known sequence.

### Example 7

### Determination of VAP-1 in clinical samples showing inflammatory response

Normal tonsil, peripheral lymph node, gut, and heart samples were freshly obtained from surgical operations, and kidney samples from organ donors. Other tissues were from autopsy samples. All these specimens were histopathologically determined to be non-inflamed. Inflamed specimens were obtained from skin punch biopsies of patients suffering from chronic dermatoses (psoriasis, atopic exzema, lichen ruber planus). Control biopsies from macroscopically uninvolved areas of the same patients were also taken. Inflamed gut specimens were from patiens with inflammatory bowel disease (Crohn's disease, ulcerative colitis) that underwent surgery for therapeutic purposes. Synovial specimens were from synovectomies.

Samples were stained with immunoperoxidase as described in Example 1. Briefly, aceton-fixed frozen sections were incubated sequntially with primary antibodies (culture supernatants or 50 µg/ml purified immunoglobulin) and with appropriate peroxidase conjugated second-stage reagents and the color reaction was developed using H₂O₂ and diaminobenzidine as the substrate. VAP-1 expression in bowel and skin samples (normal and inflamed) was analyzed by two independent readers from coded samples without knowledge of the diagnosis.

As noted in Example 1, VAP-1 is only expressed at low level in some venules of normal non-inflamed gut (Fig. 5A). In contrast, gut specimens from patients with inflammatory bowel diseases displayed a markedly increased expression of VAP-1 (Figures 5B and 5C and Table I). VAP-1 was induced both in the flat-walled venules of the lamina propria and in the HEV-like venules in organized lymphatic follicles (Peyer's patches). Also in skin, chronic inflammation was accompanied with increased synthesis of VAP-1 (Figures 6A and B). To exclude any effects of interindividial variations in the results, one sample from the dermatosis lesion and a control sample from uninvolved skin area of the same patient were simultaneously biopsied and stained. Also in these specimens, number of VAP-1 positive venules in the upper dermis was higher in the inflamed sample than in the sample from the control area. Moreover, prominent perivascular leukocyte infiltrates were constantly associated with VAP-1 positive vessels.

**TABLE I**

| Induction of VAP-1 in inflammatory bowel diseases. | | | | | | |
|---|---|---|---|---|---|---|
| Specimen | n | VAP-1 | | | | |
| | | - | + | ++ | +++ | ++++ |
| Normal | 9 | 0 | 5 | 3 | 1 | 0 |
| Crohn | 7 | 0 | 0 | 2 | 3 | 2 |
| Ulc col | 7 | 0 | 0 | 0 | 5 | 2 |
| Bowel specimens were from patients operated on for Crohn's disease, ulcerative colitis and tumors (univolved area of tumor samples represents "normal" samples). Number of VAP-1 positive venules in each sample was scored from - to ++++ as described in Materials and Methods. | | | | | | |

### Example 8

### VAP-1 mediates binding to the inflamed mucosa

In vitro frosen section assays were perfromed as described in Example 3. Monoclonal antibody 1B2 inhibited lymphocyte binding to small vessels of the lamina propria by approximately 60% (Fig. 7) in two gut samples that abundantly expressed VAP-1.

While the invention has been described in connection with specific embodiments thereof, it will be understood that it is capable of further modifications and this application is intended to cover any variations, uses, or adaptions of the invention following, in general, the principles of the invention and including such departures from the present disclosure as come within known or customary practice within the art to which the invention pertains and as may be applied to the essential features hereinbefore set forth and as follows in the scope of the appended claims.

### SEQUENCE LISTING

(1) GENERAL INFORMATION:
   (i) APPLICANT:
      (A): NAME: Jalkanen, Sirpa
      (B): STREET: Rauvolantie
      (C): CITY: PIISPANRISTI
      (E): COUNTRY: FINLAND
      (F): POSTAL CODE (ZIP) : SF-20760
   (i) APPLICANT:
      (A): NAME: Salmi, Marko
      (B): STREET: Vähä-Hämeenkatu 12 A
      (C): CITY: TURKU
      (E): COUNTRY: FINLAND
      (F): POSTAL CODE (ZIP): SF-20500
   (ii) TITLE OF INVENTION: A Novel Endothelial Cell Molecule Mediating Lymphocyte Binding in Man
   (iii) NUMBER OF SEQUENCE: 1
   (iv) CORRESPONDENCE ADDRESS:
      (A) ADDRESSEE:
         Orion Corporation, ORION-FARMOS
         PHARMACEUTICALS, Patent Department
      (B) STREET: Orionintie 1
      (C) CITY: ESPOO
      (E) COUNTRY: FINLAND
      (F) ZIP: SF-02200
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: ASCII-format
   (vi) PRIOR APPLICATION DATA:
      (A) APPLICATION NUMBER: US895354
      (B) FILING DATE: June 6, 1992
(2) INFORMATION FOR SEQ ID NO:1:
   (i) SEQUENCE CHARACTERISTICS:
      (A) LENGTH: 20 amino acids
      (B) TYPE: amino acid
      (C) STRANDEDNESS: both
      (D) TOPOLOGY: linear
   (ii) MOLECULE TYPE: peptide
   (xi) SEQUECE DESCRIPTION:SEQ ID NO:1:

Thr Glu Asp Gly Asp Met Xaa Leu Val Asn Gly Ala Ser
1               5                   10
Ala Asn Glu Gly Xaa Val Glu
    15                  20

## Claims

1. Substantially purified and isolated human endothelial vascular adhesion protein-1 (VAP-1) having a molecular weight of 90 kDa as determined by SDS-PAGE under reducing conditions and being recognizable by the monoclonal antibody obtainable by the hybridoma cell line having the accession number DSM ACC 2041.

2. The VAP-1 according to claim 1, wherein said VAP-1 binds to lymphocytes.

3. The VAP-1 according to claim 1 or 2, wherein said VAP-1 is obtainable by a process comprising binding said VAP-1 to the anti-VAP-1 antibody obtainable by the hybridoma cell line having the accession number DSM ACC 2041.

4. An anti-VAP-1 antibody, or an anti-VAP-1 binding fragment thereof, wherein said antibody or said binding fragment is specific for human endothelial VAP-1 according to any one of claims 1 to 3, and wherein said antibody does not bind to human umbilical vein endothelial cell (HUVEC) surface proteins.

5. The anti-VAP-1 antibody according to claim 4, wherein said VAP-1 antibody is a polyclonal antibody or a monoclonal antibody.

6. The anti-VAP-1 antibody according to claim 5 which is a monoclonal antibody 1B2 specifically binding to vascular adhesion protein VAP-1, obtainable by a hybridoma cell line having the accession number DSM ACC 2041.

7. A hybridoma cell line producing an anti-VAP-1 antibody according to claim 4 or 5.

8. The hybridoma cell line of claim 7, having the accession number DSM ACC 2041.

9. A cell-free composition comprising an anti-VAP-1 antibody, or an anti-VAP-1 binding fragment thereof according to any one of claims 4 to 6.

10. A method for antagonizing VAP-1-mediated binding of endothelial cells to lymphocytes, said method comprising inhibiting in vitro the VAP-1-mediated lymphocyte-endothelial cell adhesion reaction by providing amounts of an anti-VAP-1 antibody according to any one of claims 4 to 6 sufficient to block the VAP-1 endothelial cell sites that participate in said reaction.

11. A method for diagnosing a medical condition in a patient that is mediated by VAP-1-mediated binding of endothelial cells to lymphocytes, said method comprising:
(1) removing cells from said patient suspected of being VAP-1 positive cells;
(2) exposing said cells of step (1) to an anti-VAP-1 antibody or an anti-VAP-1 binding fragment thereof according to any one of claims 4 to 6;
(3) detecting anti-VAP-1 antibody or anti-VAP-1 binding fragment thereof binding to said VAP-1 positive cells; and
(4) diagnosing said medical condition on the basis of the amount of said binding.

12. The method of claim 11, wherein said medical condition is a disease selected from the group consisting of inflammation (chronic or acute), arthritis, rheumatoid arthritis, infection, dermatosis, inflammatory bowel disease, and autoimmune disease.

13. Pharmaceutical composition for use in inhibiting the VAP-1-mediated lymphocyte-endothelial cell adhesion reaction, comprising an anti-VAP-1 antibody or an anti-VAP-1 binding fragment according to any one of claims 4 to 6 and, optionally, a pharmaceutically acceptable carrier.

14. Diagnostic composition for use in diagnosing VAP-1-mediated binding of endothelial cells to lymphocytes in a patient, comprising an anti-VAP-1 antibody or an anti-VAP-1 binding fragment according to any one of claims 4 to 6 and, optionally, a carrier.

## Patentansprüche

1. Im wesentlichen gereinigtes und isoliertes menschliches endotheliales vaskuläres Adhäsions-Protein-1 (VAP-1), das ein durch SDS-PAGE unter reduzierenden Bedingungen bestimmtes Molekulargewicht von 90 kd aufweist und das durch den monoclonalen Antikörper erkannt werden kann, der von der Hybridomzellinie mit der Hinterlegungsnummer DSM ACC 2041 erhältlich ist.

2. VAP-1 nach Anspruch 1, wobei das VAP-1 an Lymphocyten bindet.

3. VAP-1 nach Anspruch 1 oder 2, wobei das VAP-1 durch ein Verfahren erhältlich ist, das die Bindung des VAP-1 an den Anti-VAP-1-Antikörper umfaßt, der von der Hybridomzellinie mit der Hinterlegungsnummer DSM ACC 2041 erhältlich ist.

4. Anti-VAP-1-Antikörper oder Anti-VAP-1-bindendes Fragment davon, wobei der Antikörper oder das bindende Fragment für menschliches endotheliales VAP-1 nach einem der Ansprüche 1 bis 3 spezifisch ist und wobei der Antikörper nicht an Oberflächenproteine von Endothelzellen menschlicher Nabelvenen (HUVEC) bindet.

5. Anti-VAP-1-Antikörper nach Anspruch 4, wobei der VAP-1-Antikörper ein polyclonaler Antikörper oder ein monoclonaler Antikörper ist.

6. Anti-VAP-1-Antikörper nach Anspruch 5, welcher der monoclonale Antikörper 1B2 ist, der spezifisch an das vaskuläre Adhäsions-Protein VAP-1 bindet, wobei dieser Antikörper von der Hybridomzellinie mit der Hinterlegungsnummer DSM ACC 2041 erhältlich ist.

7. Hybridomzellinie, die einen Anti-VAP-1-Antikörper nach Anspruch 4 oder Anspruch 5 produziert.

8. Hybridomzellinie nach Anspruch 7, welche die Hinterlegungsnummer DSM ACC 2041 hat.

9. Zellfreie Zusammensetzung, die einen Anti-VAP-1-Antikörper oder ein Anti-VAP-1-bindendes Fragment davon nach einem der Ansprüche 4 bis 6 umfaßt.

10. Verfahren zur Antagonisierung einer VAP-1-vermittelten Bindung von Endothelzellen an Lymphocyten, wobei das Verfahren die in vitro-Hemmung der VAP-1-vermittelten Adhäsionsreaktion zwischen Lymphocyten und Endothelzellen umfaßt, indem ausreichend große Mengen eines Anti-VAP-1-Antikörpers nach einem der Ansprüche 4 bis 6 bereitgestellt werden, um die VAP-1-Stellen der Endothelzellen zu blockieren, die an der Reaktion beteiligt sind.

11. Verfahren zur Diagnose eines medizinischen Zustands bei einem Patienten, der durch eine VAP-1-vermittelte Bindung von Endothelzellen an Lymphocyten vermittelt wird, wobei das Verfahren die folgenden Schritte umfaßt:
(1) Entnahme von Zellen von einem Patienten, von denen man annimmt, daß sie VAP-1-positive Zellen sind;
(2) Inkontaktbringen der Zellen von Schritt (1) mit einem Anti-VAP-1-Antikörper oder einem Anti-VAP-1-bindenden Fragment davon nach einem der Ansprüche 4 bis 6;
(3) Nachweis der Bindung des Anti-VAP-1-Antikörpers oder des Anti-VAP-1-bindenden Fragments davon an die VAP-1-positiven Zellen; und
(4) Diagnose des medizinischen Zustands aufgrund des Ausmaßes der Bindung.

12. Verfahren nach Anspruch 11, wobei der medizinische Zustand eine Erkrankung ist, die ausgewählt ist aus Entzündung (chronisch oder akut), Arthritis, rheumatoide Arthritis, Infektion, Dermatose, entzündliche Darmerkrankung und Autoimmunleiden.

13. Arzneimittel zur Verwendung für die Hemmung der VAP-1-vermittelten Adhäsionsreaktion zwischen Lymphocyten und Endothelzellen, umfassend einen Anti-VAP-1-Antikörper oder ein Anti-VAP-1-bindendes Fragment nach einem der Ansprüche 4 bis 6 und gegebenenfalls einen pharmazeutisch verträglichen Träger.

14. Diagnostische Zusammensetzung zur Verwendung für die Diagnose einer VAP-1-vermittelten Bindung von Endothelzellen an Lymphocyten bei einem Patienten, umfassend einen Anti-VAP-1-Antikörper oder ein Anti-VAP-1-bindendes Fragment nach einem der Ansprüche 4 bis 6 und gegebenenfalls einen Träger.

## Revendications

1. Protéine-1 d'adhérence vasculaire endothéliale humaine (VAP-1), essentiellement purifiée et isolée, ayant une masse moléculaire de 90 kDa déterminée par SDS-PAGE dans des conditions réductrices et reconnaissable par l'anticorps monoclonal obtenu par la lignée cellulaire hybridome ayant reçu le numéro d'enregistrement DSM ACC 2041.

2. VAP-1 selon la revendication 1, où ladite VAP-1 se lie aux lymphocytes.

3. VAP-1 selon la revendication 1 ou 2, où ladite VAP-1 peut être obtenue par un procédé comprenant, la liaison de ladite VAP-1 à l'anticorps anti-VAP-1 qui peut être obtenu par la lignée cellulaire hybridome ayant reçu le numéro d'enregistrement DSM ACC 2041.

4. Anticorps anti-VAP-1 ou un fragment liant anti-VAP-1 de celui-ci, où ledit anticorps ou ledit fragment liant de celui-ci est spécifique pour VAP-1 endothéliale humaine selon l'une quelconque des revendications 1 à 3, et où ledit anticorps ne se lie pas aux protéines de surface de la cellule endothéliale de la veine ombilicale humaine (HUVEC).

5. Anticorps anti-VAP-1 selon la revendication 4, où ledit anticorps VAP-1 est un anticorps polyclonal ou un anticorps monoclonal.

6. Anticorps anti-VAP-1 selon la revendication 5, qui est un anticorps monoclonal 1B2 se liant spécifiquement à la protéine d'adhérence vasculaire VAP-1, qui peut être obtenu par la lignée cellulaire hybridome ayant reçu le numéro d'enregistrement DSM ACC 2041.

7. Lignée de cellule hybridome produisant un anticorps anti-VAP-1 selon la revendication 4 ou 5.

8. Lignée de cellule hybridome selon la revendication 7, ayant reçu le numéro d'enregistrement DSM ACC 2041.

9. Composition exempte de cellules comprenant un anticorps anti-VAP-1 ou un fragment liant anti-VAP-1 de celui-ci, selon l'une quelconque des revendications 4 à 6.

10. Méthode permettant d'avoir une action antagoniste vis-à-vis de la liaison médiée par VAP-1 des cellules endothéliales aux lymphocytes, ladite méthode consistant à inhiber in vitro la réaction d'adhérence lymphocyte-cellule endothéliale médiée par VAP-1 en fournissant des quantités d'un anticorps anti-VAP-1 selon l'une quelconque des revendications 4 à 6 suffisantes pour bloquer les sites VAP-1 de cellules endothéliales qui participent à ladite réaction.

11. Méthode de diagnostic de l'état pathologique d'un patient, médié par la liaison médiée par VAP-1 des cellules endothéliales aux lymphocytes, ladite méthode comprenant les étapes consistant :
(1) à éliminer les cellules dudit patient susceptibles d'être des cellules VAP-1-positives ;
(2) à exposer lesdites cellules de l'étape (1) à un anticorps anti-VAP-1 ou à un fragment liant anti-VAP-1 de celui-ci selon l'une quelconque des revendications 4 à 6;
(3) à détecter la liaison de l'anticorps anti-VAP-1 ou d'un fragment liant anti-VAP-1 de celui-ci auxdites cellules VAP-1-positives ; et
(4) à diagnostiquer ledit état pathologique en se basant sur la proportion de ladite liaison.

12. Méthode selon la revendication 11, dans laquelle ledit état pathologique est une maladie appartenant au groupe formé par une inflammation (chronique ou aiguë), l'arthrite, l'arthrite rhumatoïde, une infection, une dermatose, une maladie inflammatoire des intestins et une maladie autoimmune.

13. Composition pharmaceutique destinée à être utilisée pour inhiber la réaction d'adhérence lymphocyte-cellule endothéliale médiée par VAP-1, comprenant un anticorps anti-VAP-1 ou un fragment liant anti-VAP-1 de celui-ci selon l'une quelconque des revendications 4 à 6, et éventuellement, un véhicule pharmaceutiquement acceptable.

14. Composition de diagnostic destinée à étre utilisée pour diagnostiquer la liaison médiée par VPA-1 des cellules endothéliales aux lymphocytes chez un patient, comprenant un anticorps anti-VAP-1 ou un fragment liant anti-VAP-1 de celui-ci selon l'une quelconque des revendications 4 à 6, et éventuellement, un véhicule.
